Europäisches Patentamt

European Patent Office

Office européen des brevets

(11) Publication number: **0 322 978**
**A1**

## EUROPEAN PATENT APPLICATION

(21) Application number: 88202988.7

(51) Int. Cl.⁴: **A61M 5/14**

(22) Date of filing: 21.12.88

(30) Priority: 22.12.87 NL 8703099

(43) Date of publication of application:
05.07.89 Bulletin 89/27

(84) Designated Contracting States:
DE FR GB IT NL

(71) Applicant: **CORDIS EUROPA N.V.**
**Oosteinde 8**
**NL-9301 LJ Roden(NL)**

(72) Inventor: **Kroon, Robert**
**De Wiecken 1**
**NL-9351 AX Leek(NL)**

(74) Representative: **Smulders, Theodorus A.H.J.,**
**Ir. et al**
**Vereenigde Octrooibureaux Nieuwe Parklaan**
**107**
**NL-2587 BP 's-Gravenhage(NL)**

(54) Implantable audio signalling flow monitor.

(57) A monitor which is suitable for implanting within
a human body and which signals audibly the flow of
a liquid, such as a medicament, from a source,
through the monitor, and to the dispensing site. A
liquid port is associated with the monitor through
which liquid can be introduced to the monitor. A filter
(16) may be interposed between the source and the
dispensing site to prevent the transportation of un-
wanted components, such as bacteria, to the dis-
pensing site.

FIG.2

## IMPLANTABLE AUDIO SIGNALLING FLOW MONITOR

Background and Description of the Invention

The present invention generally relates to an implantable audio signalling device. More particularly, the invention relates to an elongated fluid-confining member through which liquid material can be transported from a source for the liquid material to a dispensing location of the liquid material. The device is implantable and is useful with a substantially flexible transport tube. Uniquely, the invention provides an audio signal that denotes when a quantity of liquid passes therethrough, typically in response to the pressure created in the liquid after a patient manipulates the propelling assembly of the dispenser device located upstream of the audio signalling device.

In the medical treatment of patients, situations may occur in which it is desirable for the patient to administer to himself or herself a liquid medicament, such as a sedative, locally within the body. To that end, subcutaneously implantable dispensers are known and commercially available. An example of such a known subcutaneous, implantable dispenser is described in Harris U.S. Patent No. 4,548,607 and de Vries et al U.S. Patent No. 4,668,231 and European Patent publication No. 84201719.6. The subject matter of these publications is incorporated by reference hereinto. These types of dispensers contain or are connected with a reservoir for liquid medicament, and they include means for pumping the liquid in a controlled manner from the reservoir, through a flexible hose connected to the dispenser, and to the desired dispensing location. A patient can operate the pump percutaneously and from the exterior such as by means of a push button system. Most of the known implantable dispensers have a closed filler port, which is made from a self-sealing material, such as silicone rubber, and which is penetrable by a hollow needle, such as a hypodermic needle device. The known dispenser filler port provides access to the reservoir so that it can be refilled from the exterior and percutaneously.

The hose through which the liquid medicament is transported from the dispenser to the dispensing location generally consists of two sections. A first section is fixedly connected to the dispenser. A second section is a loose hose section. By surgical means, the loose hose section is positioned within the body of the patient in such a manner that it terminates at the location which is to receive the liquid medicament. The opposite end of the loose hose section is properly accessible to the free end of the first hose section when the latter is implanted with the dispenser. The first hose section and the second hose section are interconnected by means of a connector, which is often made of metal, to form one through-connection for the liquid medicament.

An implantable dispenser generally offers a substantial advantage to a patient in terms of the self-administration of liquid medicament. However, the patient may complain that he or she is unsure whether the manipulation of the propelling assembly of the dispenser actually has resulted in the intended displacement of liquid medicament from the reservoir.

The present invention provides a monitor which eliminates the uncertainty and anxiety which the patient experiences in self-administering liquid medicament with an implantable dispenser. Specifically, the monitor provides coacting means which emit an audible signal when a quantity of the liquid material is displaced through the transport tube. Devices according to this invention are implantable and include an inlet tube or sleeve, an outlet tube or sleeve, and a chamber for through-flow of the liquid material, whose supply end is connected to the inlet section and whose discharge end is connected to the outlet section. A control assembly is provided within the chamber and consists of a valve and an elastic wall. The valve, in the at-rest position, rests on a valve·seat thereby shutting off the connection to the inlet tube section. The valve, when not in the at-rest position - that is, when liquid material is to be transported - is lifted off the valve seat and the elastic wall of the chamber's control assembly is simultaneously displaced in a direction away from the chamber by the hydraulic pressure of the liquid material in the inlet tube section. This opens a passage for the release of the liquid material through the chamber. Accordingly, the control assembly allows a one-way flow of the liquid material.

The unique audible signalling capacity of the invention is caused by electronic circuitry, which may be battery-powered and which incorporates a sound emitter. The circuitry includes an electronic contact member located at a side remote from the chamber by means of which the electronic circuitry can be closed when the valve is lifted, thereby emitting an audible signal.

It is, accordingly, a general object of the present invention to provide a monitor which is implantable and which emits an audible signal when liquid, such as a medicament, is dispensed.

It is an object also to provide a member which is tubular and made from rigid material and constitutes a subcutaneous implantable tubular dispenser for a liquid medicament.

Another object of the invention is to provide a fluid medicament dispenser that facilitates the self-administration of a unit dosage of the medicament and which, because of the rigid construction of the monitor, may be suitable to house audible signal-emitting means for purposes of advising the user when fluid medicament is displaced through a flexible tube.

Additionally, an object of the present invention is to provide a fluid medicament dispenser that can be inserted at the connection location of hose sections, one of which is secured to a dispenser and the other of which is a loose hose section that leads to the area of the patient which is to be treated.

These and other objects of the present invention will be apparent from the following description of the invention, taken in conjunction with the accompanying drawings.

## Brief Description of the Drawings

In the course of this description, reference is made to the attached drawings, wherein:

Figure 1 is a top perspective view of a known subcutaneously implantable dispenser for a liquid medicament, such as a sedative, which dispenser is suitable for use in combination with the audio signaling device of the present invention; and

Figure 2 is a generally diagrammatic and cross-sectional view of a preferred implantable audio signalling liquid-directing device according to the present invention.

## Description of the Particular Embodiments

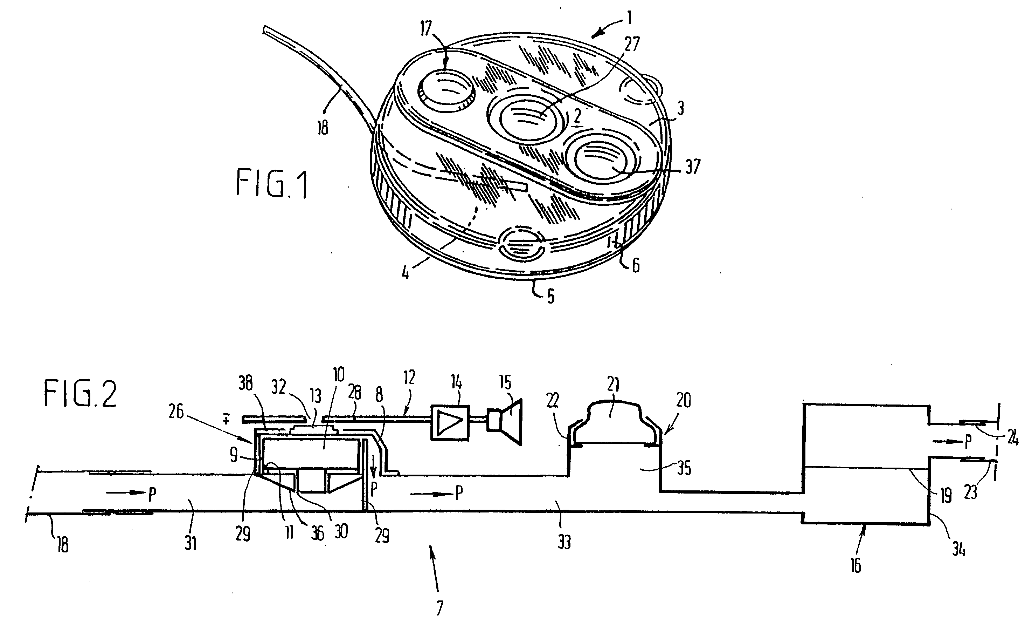

The known dispenser for liquid medicament, illustrated in Figure 1 and generally designated by reference numeral 1, has a casing which typically includes an elevated component 2 and a top wall 3, both of which are made of a relatively rigid, biocompatible polymeric material. An exemplary biocompatible material that is suitable in this regard is polyethyl sulfone. Elevated component 2 and body component 3 can be integrally molded units or can consist of two separate components firmly secured by appropriate means, such as through ultrasonic sealing or with fixed connection structures such as adhesives or screws.

The illustrated known dispenser 1 further includes a reservoir 4 for the liquid medicament, whose upper portion is formed by the body component 3, and whose bottom portion is formed by a yielding wall 5. Yielding wall 5 is, for example, suitably made of a silicone rubber that is reinforced with polyester fibers. Yielding wall 5 is secured to the body component 3 by suitable means, such as by a snap-on ring 6. Such means provides a tight-sealing and liquid-tight construction for securing the periphery of the yielding wall 5 to the body component 3.

The elevated component 2 contains members 17, 27, and 37. Member 17 is a filler port through which reservoir 4 can be filled with liquid medicament from the outside of the patient's body, typically by means of a hypodermic needle device, not shown. Member 17 includes an intermediate chamber, not shown, that can communicate with reservoir 4, but which is normally blocked by a silicone rubber shut-off valve, not shown. A resilient silicone rubber pad, not shown, which can be penetrated by, for example, a hypodermic needle device and which, after removal of the needle, is self-sealing, shuts off the upper portion of the intermediate chamber at the end shown in the drawing.

Member 27 and member 37 each consist of a resilient pad, not shown, which is made, for example, from a silicone rubber, and which is recessed and set in the elevated component 2. Embedded into the bottom of each member 27 and member 37 is a portion of a pushbutton, not shown in the drawings. Each pushbutton functions within a chamber, not shown, as a plunger. Hose 18 acts as a flexible transport tube for the liquid medicament. As an assembly for propelling the liquid, each pushbutton, each chamber associated with each member 27 and 37, and each pad is constructed and dimensioned in order to allow the pumping of liquid material from the reservoir 4 to hose 18 by the operation of the pushbuttons via the members 27 and 37. The entire dispenser 1 together with hose 18 is implanted subcutaneously within the patient.

The monitor device according to the present invention and as it is illustrated in Figure 2 includes, in part, a fluid-passing housing or connector 7, an audible signaller 26, a discharge tube 33, an optional liquid port 20, and an optional bacterial filter 16. These will now be discussed.

Connector 7 is implanted subcutaneously as is the known dispenser 1. Connector 7 is made of a rigid material or a synthetic plastic, such as polyethyl sulfone, and includes, at the end leading from the dispenser 1, a stub tube 25, or the like, to which hose 18 is connected. At its other end, connector 7 includes a stub tube 24, or the like, to which is connected a section of hose 23. While one end of the hose section 23 is connected to the stub tube 24, the other end of the section of hose 23 is disposed at that location within the patient's body where the liquid medicament is to be dosed.

Audible signaller assembly 26 is connected to the connector 7 and is composed of a chamber 9 enclosed by walls 29 and 29a and in which a valve 10 reciprocates. Chamber 9 is connected through opening 30 to the supply tube 31 for liquid medicament. Envelope or flexible cover 8, which, for example, can be made from silicone rubber, is firmly connected to the connector 7 and is dimensioned relatively to the outside dimensions of and surrounds chamber 9. The present invention also includes a contact 13, an electronic signalling circuit 12 with interruptor 32, printed circuit contact board 28, amplifier 14, and sound emitter 15.

Walls 29 further enclose envelope 8 in such a manner that a channel, through which liquid medicament can flow, is formed between the walls 29 and 29a, shown vertically-oriented in Figure 2, and envelope 8. Moreover, wall 38, shown horizontally-oriented in Figure 2, is associated with envelope 8 and is provided with the contact 13, which, for example, can be made from graphite, through which interruptor 32 of the electronic circuit 12 can be closed in a manner as indicated hereinafter.

Arrows P indicate the direction which the liquid medicament flows as a result of the operation of the dispenser 1, from reservoir 4, through hose 18, through supply tube 31, audible signaller assembly 26, discharge tube 33, stub hose 24, into hose 23, and on to the eventual dispensing location for the medicament.

A discharge sleeve or tube 33 is associated with the connector 7 and includes a liquid port 20 which comprises a housing 22 whose open top is sealed by a pad 21, which can be made of silicone rubber. As with the filler port 17 of dispenser 1, pad 21 can be pierced from the outside of the patient, typically by means of a hypodermic needle device, not shown, and be penetrated to within the space 35. Space 35 communicates with discharge tube 33. An additional quantity of liquid medicament, for example, can then be introduced directly into discharge tube 33 and eventually be supplied through hose 23 to the dispensing location. Upon removal of the piercing and penetrating device, pad 21 seals itself.

A bacterial filter 16 can be incorporated, when desired, downstream of the filler port 20 and the discharge tube 33. As illustrated, filter 16 includes a housing 34 within which a diaphragm 19 is stretched in such a manner that the entire free passage within the housing is shielded. Because diaphragm 19 is pervious to the liquid medicament, but not bacteria, the latter is retained. Bacterial filter 16 is connected through stub tube 24 to hose 23. Hose 23 terminates at its free end, not shown, at the desired dispensing location of the liquid medicament.

The operation of the connector 7 according to the present invention as shown in Figure 2 is as follows.

When a device, such as the dispenser 1 illustrated in Figure 1, is in the at-rest position, or quiescent state, and is not dispensing liquid material, the pressure which exists in the supply tube 31 of the connector 7 is at such a level that valve 10 rests with sealing ring 11 on valve seat 36. In this state, access of the supply tube 31 through opening 30 to chamber 9 is shut off, wall 38 of envelope 8 rests with contact 13 in a position so that no contact is being made, and the electronic signalling circuit 12 is interrupted forming gap 32.

When it is desired to dispense a unit dosage of fluid medicament, a user manipulates the dispenser 1, and specifically its propelling assembly, in order to supply the liquid medicament under pressure through hose 18 into supply tube 31. Under the influence of the increased pressure in the liquid, valve 10 is lifted off valve seat 36. The liquid medicament flows according to arrow P through opening 30 around valve 10 and past the inner portions of wall 29 and wall 29a into chamber 9 and discharge tube 33. When the monitor includes bacterial filter 16 in a position downstream of the audible signaller 26, the liquid medicament proceeds then through bacterial filter 16 and hose 23 to the dispensing location of the medicament. Simultaneously with the displacement of the liquid, the lifting of valve 10 lifts wall 38 of envelope 8 - because of the latter's flexible nature - and contact 13. Contact 13, when lifted in this fashion, eliminates gap 32 of the electronic circuit 12, thereby completing the circuit and causing sound emitter 15 to emit an audible signal. When liquid medicament is no longer pumped, the pressure in the liquid at the supply end of the connector 7 drops. Envelope 8 and valve 10 return again to the at-rest position.

A lithium battery, or the like, not shown, typically provides power for the electronic signalling circuit 12. The sound emitter 15 may be of the type constructed from two disc-like members and piezoelectric material with a board (which, for example, can be made of plastic) as the connecting piece, as used in a buzzer.

It will of course be understood that modifications can be made to the device as described above and shown in the drawings, without departing from the scope of the present invention. For instance, the present invention has been described with reference to the embodiment of the monitor shown in Figure 2, in which, the sequence viewed in terms of the flow direction of the liquid medicament is: audible signaller member to liquid port to bacterial filter. While this embodiment is preferred, the invention is not limited to that sequence. For instance, the bacterial filter 16 could also be arranged upstream of the audible signaller thereby providing a sequence such as: bacterial filter to signaller to liquid port, or liquid port to bacterial filter to signaller. It is also to be understood that the invention may be tubular and non-tubular or non-circular in cross section.

## Claims

1. An implantable monitor device for signalling audibly the flow of a liquid through the monitor, comprising:

(a) implantable sleeve-like housing means for receiving and directing liquid material from a source of the liquid material;

(b) upstream attachment means for providing fluid-passing communication with the source for liquid material, said attachment means being in fluid passing communication with said sleeve-like housing means;

(c) chamber means of said sleeve-like housing means for permitting controlled flow through the monitor device;

(d) coacting means for emitting an audible signal when the liquid material flows through said chamber means; and

(e) downstream attachment means for providing fluid-passing communication with a subcutaneous dispensing location.

2. The implantable monitor device according to claim 1 wherein said chamber means further comprises control means for facilitating the one-way flow of said liquid through said implantable monitor device.

3. The implantable monitor device according to claim 2 wherein the control means includes a valve which reciprocates in said chamber means to allow a one way flow of said liquid.

4. The implantable monitor device according to claim 1, wherein the sleeve-like housing means is tubular in cross section.

5. The implantable monitor device according to claim 1, wherein the sleeve-like housing means is made from a rigid and biocompatible material.

6. The implantable monitor device according to claim 4, wherein the sleeve-like housing means is made from a rigid and biocompatible material.

7. The implantable monitor device according to claim 1, further including a filter through which said liquid material passes.

8. The implantable monitor device according to claim 7, wherein said filter is downstream of said coacting means.

9. The implantable monitor device according to claim 7, wherein said filter is upstream of said coacting means.

10. The implantable monitor device according to claim 7, wherein said filter is pervious to said liquid but not to bacteria.

11. The implantable monitor device according to claim 8, wherein said filter is pervious to said liquid but not to bacteria.

12. The implantable monitor device according to claim 9, wherein said filter is pervious to said liquid but not to bacteria.

13. The implantable monitor according to claim 1, wherein said coacting means comprises an electronic signalling circuit.

14. The implantable monitor according to claim 13, wherein said electronic signalling circuit comprises a contact, a printed circuit contact board, an amplifier, and a sound emitter.

15. The implantable monitor device according to claim 1, wherein:

said upstream attachment means includes an inlet tube section and said downstream attachment means includes an outlet tube section;

said chamber means includes a supply end connected to the inlet tube section and a discharge end connected to the outlet tube section;

said coacting means includes battery-powered electronic circuitry incorporating a sound emitter;

said chamber means includes a valve accomodated within the chamber which allows the one-way passage of the liquid through the chamber means; and

said device further includes:

a valve seat, on which said valve, in the at-rest position, rests, thereby closing the connection to the inlet tube section, and off of which said valve, in the not-at-rest position, lifts under the influence of a hydraulic pressure created in the liquid in the inlet tube section, with simultaneous displacement of an elastic wall, which forms part of an envelope surrounding said chamber, in a direction away from the chamber and thereby allowing an opening to form for the passage of the liquid through the chamber; and

said elastic wall, at the side remote from the chamber, is provided with an electric contact member by means of which the electronic circuitry can be closed when the valve is lifted, thereby emitting the audible signal.

16. The implantable monitor device according to claim 15, further including a self-sealing liquid port penetrable by a hollow needle, said port being in liquid passing communication with said sleeve-like housing means.

17. An implantable monitor device according to claim 16 in which said liquid port is downstream of said chamber.

18. An implantable monitor device according to claim 17 in which said liquid port is upstream of said chamber.

19. In combination with a means for dispensing a liquid, such as a medicament, wherein the dispensing means retains said liquid material for the dispensing of liquid and wherein the improvement comprises:

(a) implantable sleeve-like housing means for receiving and directing said liquid material from said dispensing means;

(b) attachment means for providing fluid passing communication between said dispensing means and said sleeve-like housing means;

(c) chamber means within said sleeve-like housing means for permitting controlled flow through the monitor device;

(d) coacting means for emitting an audio signal when the liquid material flows through said chamber transport means; and

(e) downstream attachment means for providing fluid-passing communication with a subcutaneous dispensing location.

FIG.1

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 165 740 (PARKER HANNIFIN CORP.) <br> * Abstract * <br> --- | 1 | A 61 M 5/14 |
| A | US-A-3 389 387 (HULSE) <br> * Column 1, lines 26-30 * <br> --- | 1 | |
| A,D | EP-A-0 143 503 (CORDIS CORP.) <br> --- | 1 | |
| A,D | US-A-4 548 607 (D.L. HARRIS) <br> ----- | 1 | |

TECHNICAL FIELDS
SEARCHED (Int. Cl.4)

A 61 M

The present search report has been drawn up for all claims

| Place of search | Date of completion of the search | Examiner |
|---|---|---|
| THE HAGUE | 23-03-1989 | RAKOWICZ,J.M. |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons
........................................................................................
& : member of the same patent family, corresponding
     document

EPO FORM 1503 03.82 (P0401)